# EUROPÄISCHE PATENTSCHRIFT

(11) **EP 3 984 616 B1**
(45) Veröffentlichungstag und Bekanntmachung des Hinweises auf die Patenterteilung: **17.09.2025**
(21) Anmeldenummer: 20201624.2
(22) Anmeldetag: 13.10.2020
(51) Int. Cl.: B01D 29/23, B01D 36/00

(54) **FILTERVORRICHTUNG UND ABSCHEIDEEINRICHTUNG**
FILTER DEVICE AND SEPARATION DEVICE
DISPOSITIF DE FILTRE ET DISPOSITIF DE SÉPARATION

(43) Veröffentlichungstag der Anmeldung: 20.04.2022
(73) Patentinhaber: FAUDI Aviation GmbH, 35260 Stadtallendorf (DE)
(72) Erfinder: LAUBER, Uwe, 35440 Linden/Leihgestern (DE)
(74) Vertreter: Patentanwälte Olbricht Buchhold Keulertz

(56) Entgegenhaltungen:
- DE-A1- 102004 063 346
- DE-A1- 102018 108 986
- GB-A- 401 287
- US-A- 2 642 187
- US-A- 3 279 607
- US-A- 5 480 547
- US-A1- 2008 053 888
- US-A1- 2014 034 580

## Beschreibung

Die Erfindung betrifft eine Filtervorrichtung gemäß dem Oberbegriff von Anspruch 1, eine Abscheideeinrichtung gemäß Anspruch 11 und die Verwendung einer Filtervorrichtung nach Anspruch 12.

Filtervorrichtungen dienen dem Zurückhalten von ungewünschten Stoffen aus einem Fluid. So sind beispielsweise Koaleszenzabscheider bekannt, die dazu dienen, ein Medium aus einem zu reinigenden Medium abzuscheiden. Hierzu weisen sie als Filterelement ein Material auf, das für das abzuscheidende Medium schwerer durchdringbar ist als für das zu reinigende Medium. Auf diese Weise bilden sich innerhalb des Filterelements größer werdende Tropfen des abzuscheidenden Mediums, die sobald sie die Austrittsseite des Filterelements erreichen aufgrund ihrer Größe und einem Dichteunterschied zum zu reinigenden Medium absinken oder aufsteigen und in einem vorgesehenen Sammelraum sammelbar sind. Dieser Sammelraum kann dann entleert werden.

So ist beispielsweise aus DE 38 18 595 A1 bekannt, dass sich ein Koaleszenzabscheider dadurch herstellen lässt, dass ein gelochtes Stützrohr einen Wickelkern für eine glasfaserverstärkte Polyamidmatte ausbildet, sodass die mehrlagig auf das Stützrohr aufgewickelte Polyamidmatte ein Filterelement ausbildet. Das Stützrohr wird dann an seinen Enden mit einer ersten Endscheibe, die einen Zulauf ausbildet und einer abdichtenden zweiten Endscheibe verschlossen. Eingesetzt wird der Koaleszenzabscheider nunmehr dazu, freies Wasser aufweisenden Kohlenwasserstoff durch den Zulauf in das Stützrohr einzuleiten und von innen nach außen durch das Filterelement zu befördern. Hierbei wird das freie Wasser zurückgehalten und bildet Tropfen, die, sobald sie an der Außenfläche des Filterelements angelangen, nach unten ablaufen.

Ein ähnliches Umwickeln eines Stützkerns mit einem mattenförmigen Glasfasermaterial zur Ausbildung einer Filtervorrichtung bzw. eines Koaleszenzabscheiders wird auch in DE 21 26 080 C3 beschrieben. Zusätzlich ist hier innerhalb des Stützrohres ein Faltenfilter angeordnet, der von innen nach außen durchströmt wird und in einer Vorfilterstufe Feststoffe herausfiltert. Der Faltenfilter stützt sich an der Innenseite des Stützrohres ab. In verschiedenen Anwendungen solcher Filtervorrichtungen stellte sich nach kurzer Nutzungsdauer heraus, dass die Filterleistung stark nachlässt. Dies konnte auf Risse im Faltenfilter zurückgeführt werden. In Versuchen zur Analyse der Rissschäden wurde nunmehr festgestellt, dass der Faltenfilter bei höheren Rückströmungsgeschwindigkeiten bis zu einem Kollabieren zusammengedrückt und dabei irreparable beschädigt wird. Zu solchen Rückströmungen kann es in Abhängigkeit der Anwendung bei einem Abschalten des vorwärts gerichteten Förderstroms kommen, beispielsweise wenn das Fluid rückwärts zurück in einen tieferliegenden Tank strömt.

Aus US 3 279 607 A ist ein Faltenfilter bekannt, in dessen zylindrischen Durchgang ein Stützkörper mit der Form einer helixförmigen Feder sitzt. Diese hat die Aufgabe, den zylindrischen Durchgang aufrecht zu erhalten. GB 401 287 A lehrt bei einem ähnlichen Faltenfilter den Einsatz einer kräftigen Spiralfeder zur Abstützung des Faltenelements so, dass es gegen ein äußeres, perforiertes Rohr gehalten wird, insbesondere bei der vorgesehenen Durchströmung von außen nach innen. Weiterhin ist aus US 2 642 187 A ein Stützkörper bekannt, der entweder als schraubenförmige Windung oder als perforiertes Rohr ausgebildet ist, wobei der Stützkörper die Aufgabe hat, den Innendurchmesser eines Faltenfilters abzustützen.

Fernerhin ist von DE 10 2018 108 986 A1 ein Filterelement für einen Koaleszenzabscheider beschrieben.

Ausgehend hiervon stellte sich daher die Aufgabe, Schäden am Faltenfilter zu verhindern, um die Lebensdauer des Koaleszenzabscheiders zu erhöhen. Die Lösung soll dabei kostengünstig und zuverlässig sein, sowie die Effizienz des Koaleszenzabscheiders bzw. der Filtervorrichtung in der normalen Strömungsrichtung möglichst nicht reduzieren.

Hauptmerkmale der Erfindung sind im kennzeichnenden Teil von Anspruch 1 sowie den Ansprüchen 11 und 12 angegeben. Ausgestaltungen sind Gegenstand der Ansprüche 2 bis 10 sowie der Beschreibung.

Die Erfindung betrifft eine Filtervorrichtung, mit einem Stützrohr, das eine Innenseite und eine Außenseite aufweist, das auf der Innenseite einen Innenraum ausbildet, in den eine Eintrittsöffnung mündet, und das Durchtrittsöffnungen aufweist, die den Innenraum mit einem auf der Außenseite des Stützrohrs liegenden Umgebungsraum verbinden. Die Filtervorrichtung weist zudem ein erstes Filterelement mit einer Innenseite und einer Außenseite auf, wobei das erste Filterelement im Innenraum des Stützrohrs sowie mit seiner Außenseite benachbart zur Innenseite des Stützrohrs angeordnet ist. Dabei ist vorgesehen, dass ein Stützkörper im Innenraum des Stützrohrs sowie benachbart zur Innenseite des ersten Filterelements angeordnet ist.

Vorteilhaft hieran ist, dass sich das erste Filterelement bei einem Rückströmen erfindungsgemäß an dem Stützkörper abstützen kann. Hierdurch wird ein Kollabieren und/oder Zusammenfallen des ersten Filterelementes beim Rückströmen verhindert.

Gemäß einer näheren Ausgestaltung der Filtervorrichtung ist vorgesehen, dass sich der Stützkörper entlang des Stützrohres erstreckt, insbesondere auch entlang dem ersten Filterelement. Hierdurch kann der Stützkörper das erste Filterelement gleichmäßig stützen.

In einer speziellen Ausführungsform ist das erste Filterelement ein Faltenfilter. Solche Faltenfilter haben zwar eine recht hohe Eigenstabilität, die aber bei Überschreitung eines Grenzwertes beim Rückströmungsvolumenstrom relativ plötzlich versagt, dies meist inklusive Rissen im Bereich der Falten. Der Stützkörper kann dies zuverlässig verhindern. Derartige Faltenfilter haben Falten. Diese können sich entlang dem Stützrohr winden oder entsprechend eines Faltenbalgs quer zum Stützrohr ausgerichtet sein. Besonders bevorzugt erstrecken sich die Falten jedoch entlang dem Stützrohr, insbesondere linear. Vor allem Falten der letzten Art resultieren in einer geringen Eigenstabilität bei Querbelastungen wie dem Rückströmen, sodass der Stützkörper ein effektives Mittel zum Abstützen darstellt.

Erfindungsgemäß ist vorgesehen, dass der Stützkörper die Form einer Helix, Wendel oder Schraube aufweist. Dies ist einfach und kostengünstig herstellbar, bietet homogen verteilte Stützstellen, hat eine hohe Eigenstabilität bei geringem Gewicht und Materialeinsatz und erlaubt das Durchströmen des Innenraums in Längsrichtung. Eine Helix ist eine Kurve, die sich mit konstanter Steigung um den Mantel eines Zylinders windet.

Im Speziellen kann ein Gangwinkel des Stützkörpers größer als 20 Grad, vorzugsweise größer als 25 Grad sein. Hierdurch wird ein großer Teil der Innenseite des ersten Filterelements für das Durchströmen freigehalten. Hierzu kann auch eine Ausführung beitragen, gemäß der eine Ganghöhe des Stützkörpers wenigstens 50%, vorzugsweise wenigstens 70% des Gesamtdurchmessers des Stützkörpers beträgt.

Bei einer kostengünstigen Variante ist der Stützkörper eingängig ausgebildet. Dies ist meist strukturell hinreichend stabil für das Stützen und besonders einfach herstellbar.

Gemäß der Erfindung ist der Stützkörper eine Schraubenfeder, insbesondere eine Schraubendruckfeder. Eine solche lässt sich besonders einfach montieren. Die erfindungsgemäße Schraubenfeder verformt sich nach Montage oder Rückströmungsbelastungen elastisch in die Ausgangsform zurück. Gleichzeitig verformt sie sich bei einem Zurückströmen und reduziert so Belastungsspitzen an den Stützstellen des ersten Filterelements.

Optional kann der Stützkörper beispielsweise aus Kunststoff und/oder Metall bestehen.

Weiterhin kann die Filtervorrichtung so ausgestaltet werden, dass eine Materialquerschnittsfläche des Stützkörpers maximal 10%, bevorzugt maximal 5% der Gesamtquerschnittsfläche des Stützkörpers beträgt. Es resultiert hierdurch bei geringem Materialeinsatz ein filigraner Stützkörper, der sich geringstmöglich auf die fluiden Ströme im Innenraum des Stützrohres auswirkt.

Eine hohe Stabilität bei geringem Materialeinsatz lässt sich unter anderem auch durch eine Ausgestaltung erzielen, gemäß der der Stützkörper eine runde Materialquerschnittsfläche und/oder Gesamtquerschnittsfläche aufweist.

Besonders zum Tragen kommt das Stützelement erfindungsgemäß dadurch, dass das Stützrohr und das erste Filterelement in einem mittleren Bereich lose aneinandergrenzen und (nur) im Bereich der zwei Enden des Stützrohres zueinander abgedichtet sind. Der mittlere Bereich ist hier nämlich besonders labil bei einem Zurückströmen und wird effektiv mit dem Stützkörper gestützt.

Im Bereich der Enden des Stützrohres kann jeweils ein Verschlusselement angeordnet sein. Dann ist vorzugsweise in einem der Verschlusselemente die Eintrittsöffnung ausgebildet.

Zur mehrstufigen Behandlung eines Fluids mit der Filtervorrichtung sitzt auf der Außenseite des Stützrohrs ein zweites Filterelement.

Zur koaleszierenden Behandlung des Fluids ist dabei wenigstens eine Filterschichtlage des zweiten Filterelements hydrophob ausgebildet. Hierdurch kann beispielsweise freies Wasser aus einem Kraftstoff entzogen werden.

Alternativ besteht die Option, dass wenigstens eine Filterschichtlage des zweiten Filterelements hydrophil ist. Dies eignet sich beispielsweise zum Abscheiden von freiem Öl aus Wasser.

Außerdem weist die Filtervorrichtung erfindungsgemäß eine Schutzhülle auf, welche das Filterelement umgibt. Die Schutzhülle ist ein hydrophiles Textil, und besonders bevorzugt ein hydrophiler Baumwollstrumpf. Die Hydrophilie unterstützt ein Ablaufen von großen Tropfen des abzuscheidenden Fluids. Ein Baumwollstrumpf ist dabei besonders kostengünstig.

Die Erfindung betrifft außerdem eine Abscheideeinrichtung mit einer Filtervorrichtung, wie sie vor- und nachstehend beschrieben ist, und mit einem Filtergehäuse mit einem Innenraum, in dem die Filtervorrichtung angeordnet ist, wobei ein Gehäusezulauf in die Eintrittsöffnung der Filtervorrichtung mündet, wobei der Innenraum des Stützrohres über die Durchtrittsöffnungen mit dem Innenraum des Filtergehäuses verbunden ist, und wobei ein Gehäuseablauf aus dem Innenraum des Filtergehäuses ausmündet.

Optional kann ein Sammelvolumen unterhalb von der Filtervorrichtung in dem Filtergehäuse ausgebildet sein, wobei ein Gehäuseablauf oberhalb des Sammelvolumens aus dem Filtergehäuse ausmündet.

Schließlich betrifft die Erfindung die Verwendung einer Filtervorrichtung wie sie vor- und nachstehend beschrieben ist, wobei auf der Außenseite des Stützrohrs ein zweites Filterelement sitzt, wobei wenigstens eine Filterschichtlage des zweiten Filterelements hydrophob ist, bei der Durchströmung mit einem Fluid, das auf einem Kohlenwasserstoff basiert, wobei mit dem ersten Filterelement Feststoffe aus dem Fluid herausgefiltert werden, und wobei mit dem zweiten Filterelement freies Wasser aus dem Fluid abgeschieden wird.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:
- Fig. 1: einen Längsschnitt durch eine Filtervorrichtung; und
- Fig. 2: einen Querschnitt durch eine Filtervorrichtung.

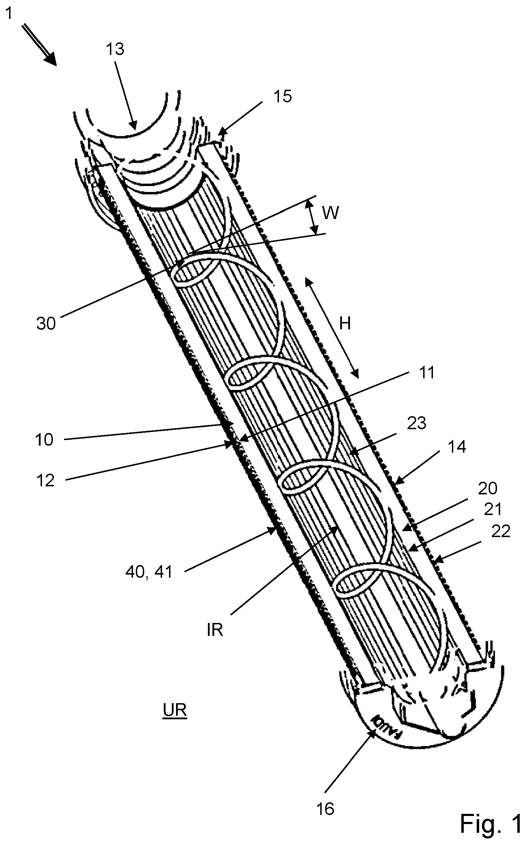

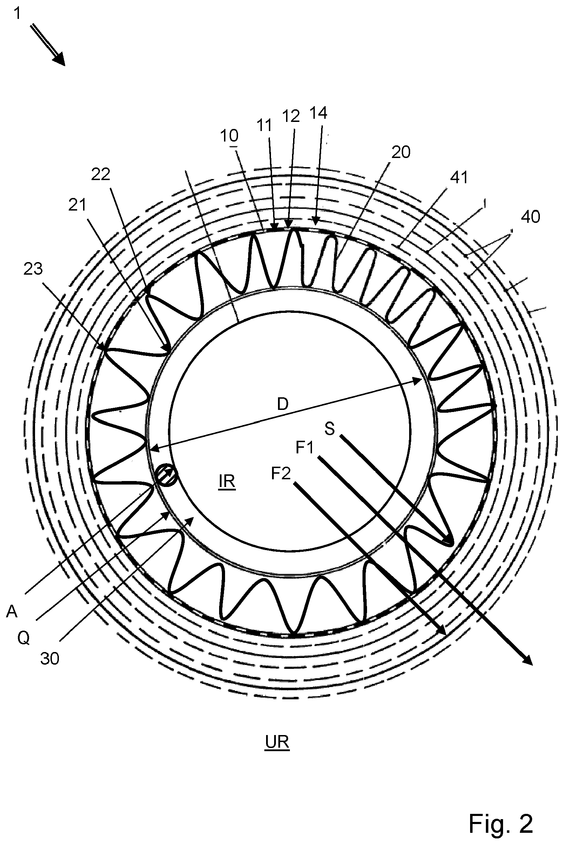

Fig. 1 zeigt einen Längsschnitt und Fig. 2 einen Querschnitt durch eine Filtervorrichtung 1. Gleiche Bezugszeichen beziehen sich daher um äquivalente Bauteile und die **Fig. 1** **und** **2** werden gemeinsam beschrieben. Die Filtervorrichtung 1 weist ein Stützrohr 10 mit einer Innenseite 11 und einer Außenseite 12 auf, wobei auf der Innenseite 11 ein Innenraum IR ausgebildet ist. Endseitig ist das Stützrohr 10 mit Verschlusselementen 15, 16 verschlossen, wobei eine Eintrittsöffnung 13 durch eines der Verschlusselemente 15 in den Innenraum IR mündet. Im Stützrohr 10 sind Durchtrittsöffnungen 14 ausgebildet, die den Innenraum IR mit einem auf der Außenseite 12 des Stützrohrs 10 liegenden Umgebungsraum UR verbinden.

Im Stützrohr 10 sitzt ein erstes Filterelement 20, insbesondere ein Faltenfilter, das eine Innenseite 21 und eine Außenseite 22 aufweist. Das erste Filterelement 20 ist mit seiner Außenseite 22 benachbart zur Innenseite 11 des Stützrohrs 10 angeordnet. Falten 23 des ersten Filterelements 20 erstrecken sich entlang dem Stützrohr 10.

Weiterhin ist ein Stützkörper 30 im Innenraum IR des Stützrohrs 10 sowie benachbart zur Innenseite 21 des ersten Filterelements 20 angeordnet. Dieser erstreckt sich entlang des Stützrohres 10. Dabei hat der Stützkörper 30 die Form einer eingängigen Helix, also einer Kurve, die sich mit konstanter Steigung um den Mantel eines Zylinders windet. Der Gangwinkel W des helixförmigen Stützkörpers 30 ist größer als 20 Grad. Die Ganghöhe H des helixförmigen Stützkörpers 30 beträgt über 50% des Gesamtdurchmessers D des Stützkörpers 30. Eine runde Materialquerschnittsfläche A des Stützkörpers 30 ist kleiner als 10% der runden Gesamtquerschnittsfläche Q des Stützkörpers 30. Im Besonderen handelt es sich bei dem Stützkörper 30 um eine Schraubendruckfeder aus Metall.

Das Stützrohr 10 und das erste Filterelement 20 liegen in einem mittleren Bereich lose aneinander und sind ausschließlich im Bereich der zwei Enden des Stützrohres 10 zueinander abgedichtet, insbesondere angrenzend zu den Verschlusselementen 15, 16.

Auf der Außenseite 12 des Stützrohrs 10 sitzt ein zweites Filterelement 40 mit mehreren Filterschichtlagen 41. Zumindest eine hiervon kann beispielsweise hydrophil oder hydrophob ausgebildet sein.

Wird die Filtervorrichtung 1 mit einem Fluid F1 aus einem Kohlenwasserstoff durchströmt, so können wie in Fig. 2 angedeutet, mit dem ersten Filterelement 20 Feststoffe S aus dem Fluid F1 herausgefiltert werden. Mit dem zweiten Filterelement 40 lässt sich freies Wasser F2 aus dem Fluid F1 abscheiden.

### Bezugszeichenliste

| | | | |
|---|---|---|---|
| 1 | Filtervorrichtung | 40 | zweites Filterelement |
| 10 | Stützrohr | 41 | Filterschichtlage |
| 11 | Innenseite | | |
| 12 | Außenseite | A | Materialquerschnittsfläche |
| 13 | Eintrittsöffnung | D | Gesamtdurchmessers |
| 14 | Durchtrittsöffnung | F1 | Fluid |
| 15 | Verschlusselement | F2 | freies Wasser |
| 16 | Verschlusselement | H | Ganghöhe |
| | | IR | Innenraum |
| 20 | erstes Filterelement | Q | Gesamtquerschnittsfläche |
| 21 | Innenseite | S | Feststoffe |
| 22 | Außenseite | UR | Umgebungsraum |
| 23 | Falte | W | Gangwinkel |
| 30 | Stützkörper | | |

## Patentansprüche

1. **Filtervorrichtung** (1), mit einem Stützrohr (10),
- das eine Innenseite (11) und eine Außenseite (12) aufweist,
- das auf der Innenseite (11) einen Innenraum (IR) ausbildet, in den eine Eintrittsöffnung (13) mündet, und
- das Durchtrittsöffnungen (14) aufweist, die den Innenraum (IR) mit einem auf der Außenseite (12) des Stützrohrs (10) liegenden Umgebungsraum (UR) verbinden,
und mit einem ersten Filterelement (20) mit einer Innenseite (21) und einer Außenseite (22),
wobei das erste Filterelement (20) im Innenraum (IR) des Stützrohrs (10) sowie mit seiner Außenseite (22) benachbart zur Innenseite (11) des Stützrohrs (10) angeordnet ist,
wobei das Stützrohr (10) und das erste Filterelement (20) in einem mittleren Bereich lose aneinandergrenzen und im Bereich der zwei Enden des Stützrohres (10) zueinander abgedichtet sind,
wobei auf der Außenseite (12) des Stützrohrs (10) ein zweites Filterelement (40) sitzt, wobei wenigstens eine Filterschichtlage (41) des zweiten Filterelements (40) hydrophob ist, und
wobei die Filtervorrichtung (1) eine Schutzhülle aufweist, die das Filterelement umgibt, wobei die Schutzhülle ein hydrophiles Textil ist,
**dadurch gekennzeichnet, dass**
ein Stützkörper (30) im Innenraum (IR) des Stützrohrs (10) sowie benachbart zur Innenseite (21) des ersten Filterelements (20) angeordnet ist, sodass sich das erste Filterelement (20) bei einem Rückströmen an dem Stützkörper (30) abstützen kann und hierdurch ein Kollabieren und/oder Zusammenfallen des ersten Filterelementes (20) beim Rückströmen verhindert wird,
wobei der Stützkörper (30) die Form einer Helix, Wendel oder Schraube aufweist,
wobei der Stützkörper (30) eine solche Schraubenfeder ist,
- die sich bei einem Zurückströmen verformt und so Belastungsspitzen an den Stützstellen des ersten Filterelements (20) reduziert, sowie
- sich nach einer Rückströmungsbelastung elastisch in die Ausgangsform zurückverformt.

2. Filtervorrichtung (1) nach Anspruch 1, **dadurch gekennzeichnet, dass** sich der Stützkörper (30) entlang des Stützrohres (10) erstreckt.

3. Filtervorrichtung (1) nach einem der Ansprüche 1 oder 2, **dadurch gekennzeichnet, dass** das erste Filterelement (20) ein Faltenfilter ist.

4. Filtervorrichtung (1) nach einem der vorhergehenden Ansprüche, **dadurch gekennzeichnet, dass** ein Gangwinkel (W) des Stützkörpers (30) größer als 20 Grad, vorzugsweise größer als 25 Grad ist.

5. Filtervorrichtung (1) nach einem der vorhergehenden Ansprüche, **dadurch gekennzeichnet, dass** eine Ganghöhe (H) des Stützkörpers (30) wenigstens 50%, vorzugsweise wenigstens 70% des Gesamtdurchmessers (D) des Stützkörpers (30) beträgt.

6. Filtervorrichtung (1) nach einem der vorhergehenden Ansprüche, **dadurch gekennzeichnet, dass** der Stützkörper (30) eingängig ausgebildet ist.

7. Filtervorrichtung (1) nach einem der vorhergehenden Ansprüche, **dadurch gekennzeichnet, dass** der Stützkörper (30) eine Schraubendruckfeder ist.

8. Filtervorrichtung (1) nach einem der vorhergehenden Ansprüche, **dadurch gekennzeichnet, dass** eine Materialquerschnittsfläche (A) des Stützkörpers (30) maximal 10%, bevorzugt maximal 5% der Gesamtquerschnittsfläche (Q) des Stützkörpers (30) beträgt.

9. Filtervorrichtung (1) nach einem der vorhergehenden Ansprüche, **dadurch gekennzeichnet, dass** der Stützkörper (30) eine runde Materialquerschnittsfläche (A) und/oder Gesamtquerschnittsfläche (Q) aufweist.

10. Filtervorrichtung (1) nach einem der vorhergehenden Ansprüche, **dadurch gekennzeichnet, dass** wenigstens eine Filterschichtlage (41) des zweiten Filterelements (40) hydrophil ist.

11. **Abscheideeinrichtung** mit einer Filtervorrichtung (1) nach einem der vorhergehenden Ansprüche und mit einem Filtergehäuse mit einem Innenraum, in dem die Filtervorrichtung (1) angeordnet ist, wobei ein Gehäusezulauf in die Eintrittsöffnung (13) der Filtervorrichtung (1) mündet, wobei der Innenraum (IR) des Stützrohres (10) über die Durchtrittsöffnungen (14) mit dem Innenraum des Filtergehäuses verbunden ist, und wobei ein Gehäuseablauf aus dem Innenraum des Filtergehäuses ausmündet.

12. **Verwendung** einer Filtervorrichtung (1) gemäß einem der Ansprüche 1 bis 10 bei der Durchströmung mit einem Fluid (F1), das auf einem Kohlenwasserstoff basiert, wobei mit dem ersten Filterelement (20) Feststoffe (S) aus dem Fluid (F1) herausgefiltert werden, und wobei mit dem zweiten Filterelement (40) freies Wasser (F2) aus dem Fluid (F1) abgeschieden wird.

## Claims

1. Filter device (1) comprising a supporting tube (10)
- which has an inner side (11) and an outer side (12),
- which on the inner side (11) forms an inner space (IR) into which an inlet opening (13) leads, and
- which has through-openings (14) connecting the inner space (IR) to a surrounding space (UR) lying on the outer side (12) of the supporting tube (10),
and comprising a first filter element (20) which has an inner side (21) and an outer side (22), the first filter element (20) being arranged in the inner space (IR) of the supporting tube (10) and with its outer side (22) adjacent to the inner side (11) of the supporting tube (10),
wherein the supporting tube (10) and the first filter element (20) loosely adjoin one another in a centre region and are sealed with respect to one another in the region of the two ends of the supporting tube (10),
wherein a second filter element (40) sits on the outer side (12) of the supporting tube (10), at least one filter layer (41) of the second filter element (40) being hydrophobic, and
wherein the filter device (1) has a protective sleeve surrounding the filter element,
wherein the protective sleeve is a hydrophilic textile, **characterized in that**
a supporting body (30) is arranged in the inner space (IR) of the supporting tube (10) and adjacent to the inner side (21) of the first filter element (20), with the result that, when backflow is occurring, the first filter element (20) can be supported against the supporting body (30) and thereby prevent collapse and/or subsidence of the first filter element (20) when backflow is occurring,
wherein the supporting body (30) has the form of a helix, a coil or a screw,
wherein the supporting body (30) is that type of helical spring
- that deforms when backflow is occurring and thus reduces loading peaks at the support points of the first filter element (20), and
- elastically deforms back into the starting form after being subjected to a backflow loading.

2. Filter device (1) according to Claim 1, **characterized in that** the supporting body (30) extends along the supporting tube (10).

3. Filter device (1) according to either of Claims 1 and 2, **characterized in that** the first filter element (20) is a pleated filter.

4. Filter device (1) according to any of the preceding claims, **characterized in that** a pitch angle (W) of the supporting body (30) is greater than 20 degrees, preferably greater than 25 degrees.

5. Filter device (1) according to any of the preceding claims, **characterized in that** a pitch (H) of the supporting body (30) is at least 50%, preferably at least 70% of the total diameter (D) of the supporting body (30).

6. Filter device (1) according to any of the preceding claims, **characterized in that** the supporting body (30) has a single-start form.

7. Filter device (1) according to any of the preceding claims, **characterized in that** the supporting body (30) is a helical compression spring.

8. Filter device (1) according to any of the preceding claims, **characterized in that** a material cross-sectional area (A) of the supporting body (30) is at most 10%, preferably at most 5% of the overall cross-sectional area (Q) of the supporting body (30).

9. Filter device (1) according to any of the preceding claims, **characterized in that** the supporting body (30) has a round material cross-sectional area (A) and/or overall cross-sectional area (Q).

10. Filter device (1) according to any of the preceding claims, **characterized in that** at least one filter layer (41) of the second filter element (40) is hydrophilic.

11. Separating apparatus comprising a filter device (1) according to any of the preceding claims and comprising a filter housing which has an inner space in which the filter device (1) is arranged, wherein a housing inflow leads into the inlet opening (13) of the filter device (1), wherein the inner space (IR) of the supporting tube (10) is connected to the inner space of the filter housing via the through-openings (14), and wherein a housing outflow leads out of the inner space of the filter housing.

12. Use of a filter device (1) according to any of Claims 1 to 10 when a hydrocarbon-based fluid (F1) is flowing therethrough, wherein solids (S) are filtered out of the fluid (F1) by means of the first filter element (20), and wherein free water (F2) is separated off from the fluid (F1) by means of the second filter element (40).

## Revendications

1. Dispositif de filtre (1), présentant un tube support (10),
- qui présente une face interne (11) et une face externe (12),
- qui forme, sur la face interne (11), un espace interne dans lequel débouche une ouverture d'entrée (13) et
- qui présente des ouvertures de passage (14) qui relient l'espace interne (IR) à un espace environnant (UR) situé sur la face externe (12) du tube support (10), et présentant un premier élément de filtre (20) présentant une face interne (21) et une face externe (22), le premier élément de filtre (20) étant agencé dans l'espace interne (IR) du tube support (10) et avec sa face externe (22) à proximité de la face interne (11) du tube support (10),
le tube support (10) et le premier élément de filtre (20) étant contigus de manière disjointe dans une zone centrale et étanches l'un par rapport à l'autre dans la zone des deux extrémités du tube support (10),
un deuxième élément de filtre (40) étant situé sur la face externe (12) du tube support (10), au moins une couche de filtre (41) du deuxième élément de filtre (40) étant hydrophobe et
le dispositif de filtre (1) présentant une gaine de protection qui entoure l'élément de filtre,
la gaine de protection étant un textile hydrophile, **caractérisé en ce que**
un corps support (30) est agencé dans l'espace interne (IR) du tube support (10) ainsi qu'à proximité de la face interne (21) du premier élément de filtre (20), de telle sorte que le premier élément de filtre (20) peut s'appuyer sur le corps support (30) lors d'un reflux et un affaissement et/ou un écroulement du premier élément de filtre (20) lors du reflux étant ainsi empêché(s),
le corps support(30) présentant la forme d'une hélice, d'un spirale ou d'une vis,
le corps support (30) étant un ressort hélicoïdal,
- qui se déforme lors d'un flux inversé et réduit ainsi les pointes de sollicitation au niveau des sites d'appui du premier élément de filtre (20) et
- qui se déforme élastiquement en retour dans la forme de départ après une sollicitation par un reflux.

2. Dispositif de filtre (1) selon la revendication 1, **caractérisé en ce que** le corps support (30) s'étend le long du tube support (10).

3. Dispositif de filtre (1) selon l'une des revendications 1 ou 2, **caractérisé en ce que** le premier élément de filtre (20) est un filtre plissé.

4. Dispositif de filtre (1) selon l'une des revendications précédentes, **caractérisé en ce qu'**un angle de filet (W) du corps support (30) est supérieur à 20°, de préférence supérieur à 25°.

5. Dispositif de filtre (1) selon l'une des revendications précédentes, **caractérisé en ce qu'**une hauteur de filet (H) du corps support (30) représente au moins 50%, de préférence au moins 70% du diamètre total (D) du corps support (30).

6. Dispositif de filtre (1) selon l'une des revendications précédentes, **caractérisé en ce que** le corps support (30) est réalisé avec un seul filet.

7. Dispositif de filtre (1) selon l'une des revendications précédentes, **caractérisé en ce que** le corps support (30) est un ressort hélicoïdal de compression.

8. Dispositif de filtre (1) selon l'une des revendications précédentes, **caractérisé en ce qu'**une surface transversale (A) du matériau du corps support (30) représente au maximum 10%, de préférence au maximum 5% de la surface transversale totale (D) du corps support (30).

9. Dispositif de filtre (1) selon l'une des revendications précédentes, **caractérisé en ce que** le corps support (30) présente une surface transversale (A) du matériau et/ou une surface transversale totale (Q) ronde.

10. Dispositif de filtre (1) selon l'une des revendications précédentes, **caractérisé en ce qu'**au moins une couche de filtre (41) du deuxième élément de filtre (40) est hydrophile.

11. Installation de séparation présentant un dispositif de filtre (1) selon l'une des revendications précédentes et présentant un boîtier de filtre présentant un espace interne, dans lequel le dispositif de filtre (1) est agencé, une alimentation de boîtier débouchant dans l'ouverture d'entrée (13) du dispositif de filtre (1), l'espace interne (IR) du tube support (10) étant relié par l'intermédiaire des ouvertures de passage (14) à l'espace interne du boîtier de filtre et une évacuation de boîtier sortant hors de l'espace interne du boîtier de filtre.

12. Utilisation d'un dispositif de filtre (1) selon l'une des revendications 1 à 10 lors de l'écoulement d'un fluide (F1), qui est basé sur un hydrocarbure, à travers celui-ci, le premier élément de filtre (20) éliminant par filtration des solides (S) du fluide (F1) et le deuxième élément de filtre (40) séparant l'eau libre (F2) du fluide (F1).
